# EUROPEAN PATENT APPLICATION

(11) **EP 4 494 543 A1**
(43) Date of publication of application: **22.01.2025**
(21) Application number: 24189445.0
(22) Date of filing: 18.07.2024
(51) Int. Cl.: A61B 1/00, H01H 13/702, H03K 17/96, H01H 13/70

(54) **DEFLECTION-SENSED BUTTON PRESS DETECTION SYSTEM**

(30) Priority: 19.07.2023 US 202363514557 P
(71) Applicant: Stryker Corporation, Portage, MI 49002-9711 (US)
(72) Inventor: TRAN, Levey Trac, Kalamazoo, Michigan, 49002 (US); MITCHEL, West GarrettL, Kalamazoo, Michigan, 49002 (US); FEINGOLD, Benjamin Hyman, Kalamazoo, Michigan, 49002 (US); HUYNH, Eric Charles, Kalamazoo, Michigan, 49002 (US)
(74) Representative: V.O.

(57) **Abstract**

Disclosed herein are systems and methods that include a keypad that detects button presses using deflection sensing. The keypad may be a keypad of a camera such as an endoscopic camera head. A button press may be detected based on an amount of deflection of a material. The deflection of the material may be caused by force from a user's finger on a button of the keypad. The material may be located proximate to a sensor, which outputs a sensor signal indicating the amount of deflection of the material. Aspects of the disclosure comprise a controller that identifies an amount of deflection, compares the amount of deflection to a signal threshold, and determines whether a button has been pressed based on the comparison.

## Description

### CROSS-REFERENCE TO RELATED APPLICATIONS

This application claims the benefit of U.S. Provisional Application No. 63/514,557, filed July 19, 2023, the entire contents of which are hereby incorporated by reference herein.

### FIELD

The present invention relates to button press detection, particularly for an endoscopic system.

### BACKGROUND

An endoscope is a surgical tool designed to be placed inside a human body in order to provide a view of the interior portion of the human body. In endoscopic surgery, the endoscope is placed in the human body at the location where it is necessary to perform a surgical procedure. Other surgical instruments are placed in the human body at the surgical site. The user (e.g., surgeon, medical staff, etc.) views the surgical site through the endoscope in order to assess the interior portion of the human body and to manipulate other surgical instruments to perform the desired surgical procedure. The development of endoscopes and their companion surgical instruments has made it possible to perform minimally invasive surgery that eliminates the need to make a large incision in the human body to gain access to the surgical site. Instead, during endoscopic surgery, small openings, called portals, are formed. One advantage of performing endoscopic surgery is that, since the portions of the human body that are cut are smaller, the portions of the human body that need to heal after the surgery are also small. Still another advantage of endoscopic surgery is that it exposes less of the internal tissue of the human body to the open environment. This minimal opening of the human body lessens the extent to which the internal tissue and organs of the human body are open to infection.

During endoscopic surgery, the user may activate one or more functions of the endoscopic system using buttons on a keypad. For example, the user may depress a button for activating a light source or for capturing a picture of the interior portion of the human body being assessed. The user may need to be able to reliably control the endoscopic system, such as reliably activate and deactivate the light source of the endoscope, reliably capture pictures or videos, etc. Any complications with controlling the endoscopic system may cause frustration to the user and create an unsafe situation because the user (e.g., surgeon, medical staff, etc.) may be required to perform the surgical procedure without complete functionality. Button presses must be accurately detected, and phantom button presses (*e.g*., when a button has not been pressed, but the button press detection system registers a button press; or when a button has been pressed, but the button press detection system does not register the button press) must be minimized. In some aspects, the keypad may need to be sterilized, *e.g*., via an autoclaving process that uses a gas or liquid. The gas or liquid may reach the electronics of the keypad through openings of the keypad, causing damage to the electronics of the keypad. In such instances, it may be beneficial for there to be no openings in the keypad.

### SUMMARY

According to various aspects, systems and methods include a keypad that detects button presses using deflection sensing. The keypad may be a keypad of a camera such as an endoscopic camera head. A button press may be detected based on an amount of deflection of a material. The deflection of the material may be caused by force from a user's finger on a button of the keypad. The material may be located proximate to a sensor, which outputs a sensor signal indicating the amount of deflection of the material. Aspects of the disclosure comprise a controller that identifies an amount of deflection, compares the amount of deflection to a signal threshold, and determines whether a button has been pressed based on the comparison.

According to some examples, a system comprises a camera head, the system comprises: a button; a material; a sensor configured to output a sensor signal indicating an amount of deflection of the material proximate to the sensor; and a controller configured to identify a value of the sensor signal being greater than a signal threshold, wherein the value of the sensor signal being greater than the signal threshold indicates a button press.

In any of the examples, the sensor is an inductive sensor, a capacitive sensor, or a piezo sensor.

In any of the examples, the controller is further configured to:
register the button press in accordance with the value of the sensor signal being greater than the signal threshold.

In any of the examples, the controller is further configured to:
start a timer in accordance with the identification of the value of the sensor signal being greater than the signal threshold.

In any of the examples, the controller is further configured to: start a timer in accordance with the identification of the value of the sensor signal being greater than the signal threshold; and register the button press in accordance with an elapsed time of the timer being greater than a time threshold.

In any of the examples, the controller is further configured to: determine a position of the button based on the sensor signal.

In any of the examples, the button comprises a plurality of positions, each of the plurality of positions determined based on the sensor signal.

In any of the examples, the button comprises a plurality of positions, each of the plurality of positions determined based on the sensor signal, wherein the plurality of positions comprises an initial position and a final position.

In any of the examples, the controller is further configured to: determine a position of the button based on the sensor signal, wherein the position of the button comprises an initial position and a final position; and wherein the value of the sensor signal being greater than the signal threshold occurs in accordance with the button being between the initial position and the final position.

In any of the examples, the controller is further configured to: identify a second value of the sensor signal being less than a second signal threshold, wherein the second value of the sensor signal being less than the second signal threshold indicates a button release.

In any of the examples, the controller is further configured to: identify a second value of the sensor signal being less than a second signal threshold; and register the button press in accordance with a duration being less than a duration threshold corresponding to a type of button press, wherein the duration is between the identification of the value of the sensor signal being greater than the signal threshold and the identification of the second value of the sensor signal being less than the second signal threshold.

In any of the examples, the controller is further configured to: identify a second value of the sensor signal being less than a second signal threshold; and register the button press as a short press in accordance with a duration being shorter than a short press duration threshold, wherein the duration is between the identification of the value of the sensor signal being greater than the signal threshold and the identification of the second value of the sensor signal being less than the second signal threshold.

In any of the examples, the controller is further configured to: identify a second value of the sensor signal being less than a second signal threshold; and register the button press as a short press in accordance with a duration being shorter than 370 milliseconds, wherein the duration is between the identification of the value of the sensor signal being greater than the signal threshold and the identification of the second value of the sensor signal being less than the second signal threshold.

In any of the examples, the controller is further configured to: identify a second value of the sensor signal being less than a second signal threshold; and register the button press as a long press in accordance with a duration being longer than a long press duration threshold, wherein the duration is between the identification of the value of the sensor signal being greater than the signal threshold and the identification of the second value of the sensor signal being less than the second signal threshold.

In any of the examples, the controller is further configured to: identify a second value of the sensor signal being less than a second signal threshold; and register the button press as a long press in accordance with a duration longer than 470 milliseconds, wherein the duration is between the identification of the value of the sensor signal being greater than the signal threshold and the identification of the second value of the sensor signal being less than the second signal threshold.

In any of the examples, the button provides tactile feedback in accordance with the button press being registered.

In any of the examples, the button provides tactile feedback at a final position of the button.

In any of the examples, the system further comprises: a plurality of buttons, wherein each of the plurality of buttons is associated with a unique sensor signal.

In any of the examples, the system further comprises: a plurality of buttons, wherein each of the plurality of buttons is associated with a unique signal threshold.

In any of the examples, the system comprises a first button associated with a first sensor signal and a second button associated with a second sensor signal, wherein the controller further is configured to: compare the first sensor signal to the signal threshold; compare the second sensor signal to the signal threshold; and determine whether the button press corresponds to the first button, the second button, or a multi-button press based on the comparisons.

In any of the examples, the system comprises a first button associated with a first sensor signal and a second button associated with a second sensor signal, wherein the controller further is configured to: compare the first sensor signal to the signal threshold; compare the second sensor signal to a second signal threshold; and determine whether the button press corresponds to the first button, the second button, or a multi-button press based on the comparisons.

In any of the examples, the amount of deflection is between 5-200 microns.

In any of the examples, the system is an endoscopic system.

In any of the examples, the system further comprises: a camera enclosure, wherein the camera head is located inside the camera enclosure.

In any of the examples, the system further comprises: a camera enclosure comprising circuitry for the camera head.

In any of the examples, the system further comprises: a camera enclosure comprising circuitry for the camera head, wherein the circuitry is located in the camera enclosure.

In any of the examples, the material is a continuous piece of material.

In any of the examples, the material forms the button.

In any of the examples, the material is a continuous piece of material that forms a plurality of buttons.

In any of the examples, the material comprises aluminum.

In any of the examples, the material comprises one or more raised features corresponding to one or more buttons.

In any of the examples, the material comprises one or more raised features corresponding to one or more buttons, and wherein a thickness of the material at locations of the one or more raised features is greater than a thickness of the material at locations of non-raised features.

In any of the examples, the material comprises one or more raised features corresponding to one or more buttons, and wherein a top surface of a keypad of the one or more buttons is not flat.

In any of the examples, the material comprises one or more raised features corresponding to one or more buttons, and wherein a top surface of a keypad of the one or more buttons is flat.

In any of the examples, the material comprises one or more recessed features corresponding to one or more buttons.

In any of the examples, the material comprises one or more recessed features corresponding to one or more buttons, wherein a thickness of the material at locations of the one or more recessed features is less than a thickness of the material at locations of non-recessed features.

In any of the examples, the material comprises one or more raised features corresponding to one or more buttons, and wherein a top surface of a keypad of the one or more buttons is not flat.

In any of the examples, the material comprises one or more raised features corresponding to one or more buttons, and wherein a top surface of a keypad of the one or more buttons is flat.

In any of the examples, a top surface of a keypad of the button is not flat.

In any of the examples, a top surface of a keypad of the button is flat.

In any of the examples, the system further comprises: a keypad comprising a plurality of buttons located on a plurality of planes.

In any of the examples, the system further comprises: a keypad comprising a plurality of buttons located on a plurality of planes, wherein the plurality of planes comprises different planes that form a geodesic dome.

In any of the examples, the system further comprises: one or more side buttons located on one or more sides of the camera head.

In any of the examples, the button is associated with a first time threshold, the system further comprising: a side button associated with a second time threshold.

In any of the examples, the button is associated with a first time threshold, the system further comprising: a side button associated with a second time threshold, wherein the second time threshold is longer than the first time threshold.

In any of the examples, the system further comprises: a spacer located between the sensor and the material.

In any of the examples, the system further comprises: air or a non-conductive material located between the sensor and the material.

According to some examples, a method of detecting a button press of a button on a camera head comprises: receiving a sensor signal from a sensor, wherein the sensor signal is indicating an amount of deflection of a material proximate to the sensor; identifying a value of the sensor signal being greater than a signal threshold, wherein the value of the sensor signal being greater than the signal threshold indicates the button press.

In any of the examples, the amount of deflection of the material is associated with a strength of an electromagnetic field of the sensor.

In any of the examples, the amount of deflection of the material is associated with an amount of capacitive coupling.

In any of the examples, the amount of deflection of the material is associated with an amount of stretching of the sensor.

In any of the examples, the method further comprises: registering the button press in accordance with the value of the sensor signal being greater than the signal threshold.

In any of the examples, the method further comprises: starting a timer in accordance with the identification of the value of the sensor signal being greater than the signal threshold.

In any of the examples, the method further comprises: starting a timer in accordance with the identification of the value of the sensor signal being greater than the signal threshold; and registering the button press in accordance with an elapsed time of the timer being greater than a time threshold.

In any of the examples, the method further comprises: determining a position of the button based on the sensor signal.

In any of the examples, the method further comprises: determining a position of the button based on the sensor signal, wherein the position of the button comprises an initial position and a final position.

In any of the examples, the method further comprises: determining a position of the button based on the sensor signal, wherein the position of the button comprises an initial position and a final position; and wherein the value of the sensor signal being greater than the signal threshold occurs in accordance with the button being between the initial position and the final position.

In any of the examples, the method further comprises: identifying a second value of the sensor signal being less than a second signal threshold, wherein the second value of the sensor signal being less than the second signal threshold indicates a button release.

In any of the examples, the method further comprises: identifying a second value of the sensor signal being less than a second signal threshold; and registering the button press in accordance with a duration being less than a duration threshold corresponding to a type of button press, wherein the duration is between the identification of the value of the sensor signal being greater than the signal threshold and the identification of the second value of the sensor signal being less than the second signal threshold.

In any of the examples, the method further comprises: identifying a second value of the sensor signal being less than a second signal threshold; and registering the button press as a short press in accordance with a duration being shorter than a short press duration threshold, wherein the duration is between the identification of the value of the sensor signal being greater than the signal threshold and the identification of the second value of the sensor signal being less than the second signal threshold.

In any of the examples, the method further comprises: identifying a second value of the sensor signal being less than a second signal threshold; and registering the button press as a short press in accordance with a duration being shorter than 370 milliseconds, wherein the duration is between the identification of the value of the sensor signal being greater than the signal threshold and the identification of the second value of the sensor signal being less than the second signal threshold.

In any of the examples, the method further comprises: identifying a second value of the sensor signal being less than a second signal threshold; and registering the button press as a long press in accordance with a duration being longer than a long press duration threshold, wherein the duration is between the identification of the value of the sensor signal being greater than the signal threshold and the identification of the second value of the sensor signal being less than the second signal threshold.

In any of the examples, the method further comprises: identifying a second value of the sensor signal being less than a second signal threshold; and registering the button press as a long press in accordance with a duration being longer than 470 milliseconds, wherein the duration is between the identification of the value of the sensor signal being greater than the signal threshold and the identification of the second value of the sensor signal being less than the second signal threshold.

In any of the examples, the method further comprises: providing tactile feedback when the button press is registered.

In any of the examples, the method further comprises: providing tactile feedback at a final position of the button.

In any of the examples, the camera head comprises a plurality of buttons, each of the plurality of buttons is associated with independent receiving and identifying steps.

In any of the examples, the camera head comprises a first button associated with a first sensor signal and a second button associated with a second sensor signal, the method further comprising: comparing the first sensor signal to the signal threshold; comparing the second sensor signal to the signal threshold; and determining whether the button press corresponds to the first button, the second button, or a multi-button press based on the comparisons.

In any of the examples, the camera head comprises a first button associated with a first sensor signal and a second button associated with a second sensor signal, the method further comprising: comparing the first sensor signal to the signal threshold; comparing the second sensor signal to a second signal threshold; and determining whether the button press corresponds to the first button, the second button, or a multi-button press based on the comparisons.

In any of the examples, the amount of deflection is between 5-200 microns.

In any of the examples, the camera head is included in an endoscopic system.

It will be appreciated that any of the variations, aspects, features, and options described in view of the systems apply equally to the methods and vice versa. It will also be clear that any one or more of the above variations, aspects, features, and options can be combined.

### BRIEF DESCRIPTION OF THE FIGURES

The invention will now be described, by way of example only, with reference to the accompanying drawings, in which:
FIG. 1 illustrates an example endoscopic system, according to some aspects.
FIGS. 2A and 2B illustrate views of an example keypad of a camera head, according to some aspects.
FIG. 3A illustrates cross-sectional views of a user pushing a button, causing the material to move by a deflection, according to some aspects.
FIG. 3B illustrates cross-sectional views of a button comprising a material that separates the sensor from another material, according to some aspects.
FIG. 3C illustrates cross-sectional views of a button where material is not located between the material and the sensor, according to some aspects.
FIG. 4A illustrates a flowchart of an example method for detecting a button press on a keypad of a camera head, according to some aspects.
FIG. 4B illustrates a flowchart of an example method for detecting multiple button presses on a keypad of a camera head, according to some aspects.
FIGS. 5A and 5B illustrate planar and cross-sectional views of an example keypad comprising raised buttons, according to some aspects.
FIG. 5C illustrates a cross-sectional view of example buttons comprising recessed features, according to some aspects.
FIGS. 6A and 6B illustrate top and cross-sectional views, respectively, of an example keypad comprising different regions of different thicknesses, according to some aspects.
FIG. 6C illustrates a cross-sectional view of an example having a keypad having a top surface that is flat, while also having different regions of different thicknesses, according to some aspects.
FIGS. 7A and 7B illustrate cross-sectional and top views of an example keypad comprising sensors located proximate to the locations under the buttons, according to some aspects.
FIGS. 8A and 8B illustrate planar and cross-sectional views of an example keypad comprising buttons with top surfaces on a plurality of planes, according to some aspects.
FIG. 8C illustrates a top view of an example PCB comprising sensors for a keypad, according to some aspects.
FIGS. 9A and 9B illustrate planar and side views, respectively, of a camera head comprising a side button, according to some aspects.
FIG. 10 illustrates an example computing system, according to some aspects.

### DETAILED DESCRIPTION

Reference will now be made in detail to implementations and various aspects and variations of systems and methods described herein. Although several example variations of the systems and methods are described herein, other variations of the systems and methods may include aspects of the systems and methods described herein combined in any suitable manner having combinations of all or some of the aspects described.

Systems and methods according to the principles described herein detect button presses based on a deflection of a material proximate to a sensor meeting deflection criteria, such as, the amount of deflection being greater than a signal threshold. The disclosed buttons comprise one or more sensors, a material, a printed circuit board, and one or more controllers. The sensor signal output from a sensor is based on the deflection of the material proximate to the sensor. A higher sensor signal occurs when the material is located closer to the sensor, thereby having a higher amount of deflection due to, *e.g*., the button being pressed. A lower sensor signal occurs when the material is further away from the sensor, thereby having a lower amount of deflection due to, *e.g*., the button not being pressed.

In the following description, it is to be understood that the singular forms "a," "an," and "the" used in the following description are intended to include the plural forms as well, unless the context clearly indicates otherwise. It is also to be understood that the term "and/or" as used herein refers to and encompasses any and all possible combinations of one or more of the associated listed items. It is further to be understood that the terms "includes, "including," "comprises," and/or "comprising," when used herein, specify the presence of stated features, integers, steps, operations, elements, components, and/or units but do not preclude the presence or addition of one or more other features, integers, steps, operations, elements, components, units, and/or groups thereof.

Certain aspects of the present disclosure include process steps and instructions described herein in the form of an algorithm. It should be noted that the process steps and instructions of the present disclosure could be embodied in software, firmware, or hardware and, when embodied in software, could be downloaded to reside on and be operated from different platforms used by a variety of operating systems. Unless specifically stated otherwise as apparent from the following discussion, it is appreciated that, throughout the description, discussions utilizing terms such as "processing," "computing," "calculating," "determining," "displaying," "generating," or the like, refer to the action and processes of a computer system, or similar electronic computing device, that manipulates and transforms data represented as physical (electronic) quantities within the computer system memories or registers or other such information storage, transmission, or display devices.

The present disclosure in some examples also relates to a device for performing the operations herein. This device may be specially constructed for the required purposes, or it may comprise a general-purpose computer selectively activated or reconfigured by a computer program stored in the computer. Such a computer program may be stored in a non-transitory, computer readable storage medium, such as, but not limited to, any type of disk, including floppy disks, USB flash drives, external hard drives, optical disks, CD-ROMs, magnetic-optical disks, read-only memories (ROMs), random access memories (RAMs), EPROMs, EEPROMs, magnetic or optical cards, application specific integrated circuits (ASICs), or any type of media suitable for storing electronic instructions, and each coupled to a computer system bus. Furthermore, the computers referred to in the specification may include a single processor or may be architectures employing multiple processor designs for increased computing capability. Suitable processors include central processing units (CPUs), graphical processing units (GPUs), field-programmable gate arrays (FPGAs), and ASICs.

The methods, devices, and systems described herein are not inherently related to any particular computer or other apparatus. Various general-purpose systems may also be used with programs in accordance with the teachings herein, or it may prove convenient to construct a more specialized apparatus to perform the required method steps. The required structure for a variety of these systems will appear from the description below. In addition, the present invention is not described with reference to any particular programming language. It will be appreciated that a variety of programming languages may be used to implement the teachings of the present invention as described herein.

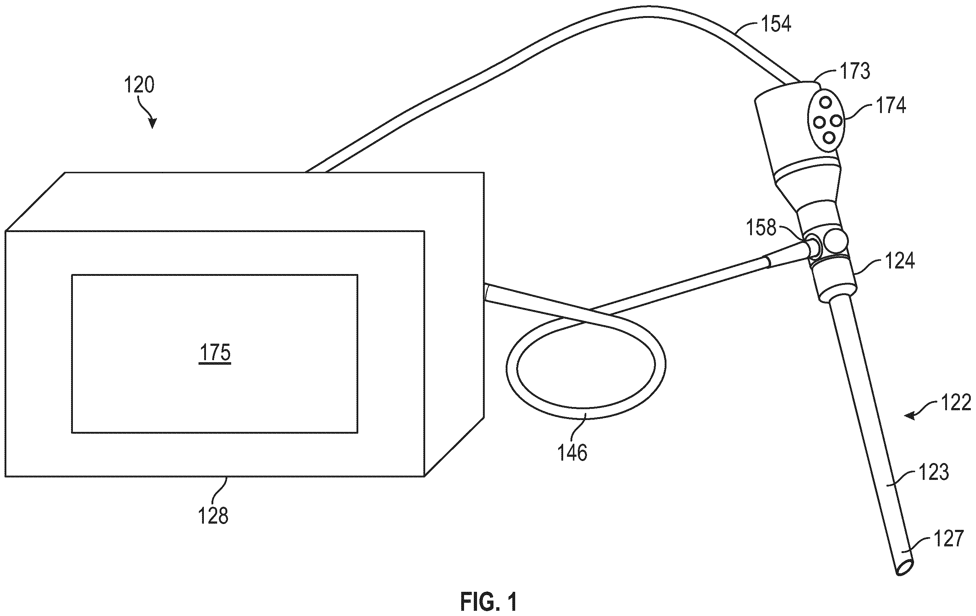

FIG. 1 illustrates an example endoscopic system, according to some aspects. The endoscopic system 120 includes an endoscope 122, transmission cables 146 and 154, and a control unit 128. The endoscope 122 is an elongated and generally hollow shaft 123 with a distal end 127 configured for insertion within an interior portion of a human body. The hollow shaft 123 also has a proximal end 124 for mounting a camera head 173. The camera head 173 provides a viewing port through which a camera allows the user to view the surgical field (for example, through a connection between a viewing port, a digital camera, and a display screen 175). The camera head 173 comprises a keypad 174, which includes a plurality of buttons. The plurality of buttons, when pressed, transmits a signal through cable 154, allowing the user to activate one or more functions of the endoscopic system 120. A light port 158 may be connected with a light source to selectively transmit light to a target via the endoscope 122.

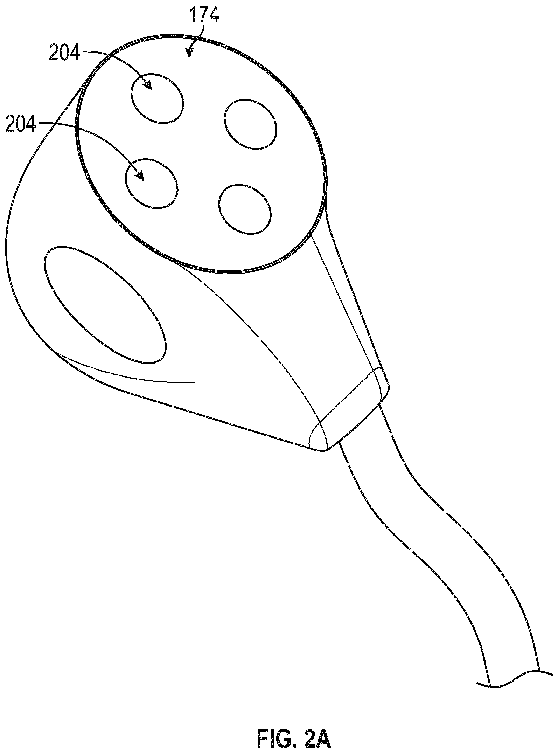

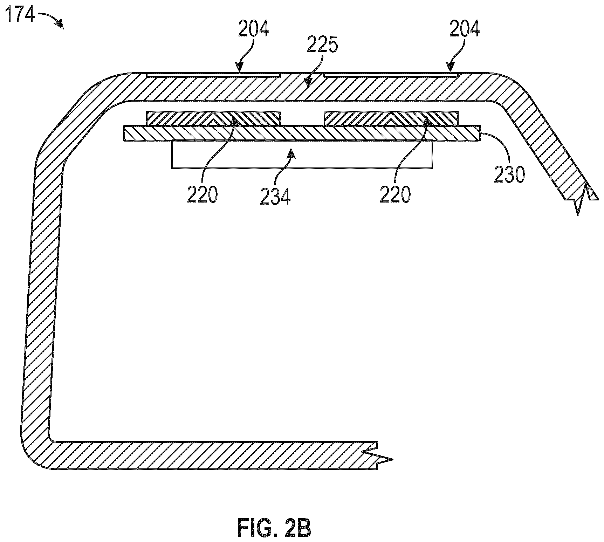

FIG. 2A and 2B illustrate views of an example keypad 174 of a camera head 173, according to some aspects. As shown in FIG. 2A, the keypad 174 comprises one or more buttons 204 for allowing a user (*e.g*., surgeon, medical staff, etc.) to activate one or more functions of the endoscopic system 120. The keypad 174 comprises any number of buttons 204 including, but not limited to, 1, 2, 3, 4, 5, 6, etc.

As shown in the cross-sectional view of FIG. 2B, the buttons 204 include one or more sensors 220 and a material 225. In some aspects, the material 225 may comprise a solid material that is rigid enough such that it does not deform when a certain amount of pressure (*e.g*., applied by a user's finger; *e.g.,* 1-10 lbf/in²) is applied, but has enough elasticity to deflect. Example materials include, but are not limited to, aluminum, stainless steel, carbon-reinforced polymer, polymer, titanium, and the like. The material 225 can be a continuous piece of material (*e.g*., no openings, seams, or gaps) that form the outer surface of the keypad 174, for example. The outer surface may be the surface that interfaces with the user's finger when pressing a button 204. In some aspects, the continuous piece of material may form the buttons 204. A homogenous button interface may be created with a continuous piece of material 225 forming a plurality of buttons 204.

A button 204 comprises a sensor 220 located below the material 225. In some aspects, the material 225 may be located between an outer surface of the keypad 174 and the sensor 220. In some aspects, the outer surface of the keypad 174 may comprise at least a portion of material 225. The sensor 220 operates based on deflection sensing. In some aspects, the number of buttons 204 may be equal to the number of sensors 220. A button press from, *e.g.,* the user's finger causes the material 225 to move toward the sensor 220. A button release from, *e.g.,* the user's finger causes the material 225 to move away from the sensor 220. The button 204 may have a plurality of positions comprising an initial position and a final position. In some aspects, the button release may occur when the button 204 is in the initial position, and the button press may occur when the button is between the initial position and the final position (including being at the final position). The sensor 220 measures the amount of deflection of the material 225 proximate to the sensor 220, and outputs a sensor signal corresponding to the measured amount of deflection. The sensor signal may comprise a voltage, current, frequency induction, capacitance, strain, etc. In some aspects, the sensor signal may be an analog signal or a digital signal. A controller receives the sensor signal from the sensor. In some aspects, the controller may determine the position of the button 204 based on the sensor signal.

The amount of deflection from the material 225 may be based on the amount of force from the user's finger (including no force). For example, a high amount of deflection may be measured when the user applies a high amount of force (*e.g.,* the user is pushing the button 204). The high amount of force may cause a higher sensor signal output due to the amount of deflection causing the material 225 to be located closer to a sensor 220. For example, the sensor signal output from the sensor 220 may be highest when the material 225 has reached its maximum amount of deformation (*e.g*., the material 225 is locally contacting the sensor 220) and the button 204 is in the final position. In some aspects, a controller registers a button press when the sensor signal is within a high sensor signal range (including the highest sensor signal). Aspects of the disclosure comprise the amount of deflection for a button press being on the order of microns (*e.g*., 5-200 microns).

A low amount of deflection may be measured when the user applies a low amount of force (*e.g*., the user is releasing the button 204 or not pushing the button 204 at all). The low amount of force may cause a lower amount of deflection (including no deflection) due to the material 225 being located further from the sensor 220. For example, the sensor signal output from the sensor 220 may be lowest when the material 225 is at its minimum amount of deformation (*e.g*., the material 225 is not deformed at all) and the button 204 is in the initial position. In some aspects, a controller does not register a button press when the sensor signal is within a low sensor signal range (including the lowest sensor signal).

The keypad 174 may comprise circuitry, such as a printed circuit board (PCB) 230. In some aspects, the PCB 230 may comprise one or more sensors 220. The sensor signal from a sensor 220 may be communicated to a controller. The PCB 230 may comprise the controller, wires, and/or any other circuitry suitable for communicating the sensor signal to the controller. The controller may receive one or more sensor signals and may determine whether one or more buttons 204 have been pressed and/or released based on the sensor signal(s). The functions associated with a button press and/or a button release comprise changing the camera head settings, such as the brightness of the light source, the operation mode such as picture capture mode or video capture mode, activating a component such as a light source, etc. In some aspects, the camera head comprises a plurality of buttons 204. At least two of the plurality of buttons may be associated with independent signals for the sensor signals and independent receiving and identifying steps. For example, the keypad 174 of the camera head 173 may comprise four buttons 204, and the controller may receive four separate signals, one for each button 204. In some aspects, each of the plurality of buttons is associated with a unique sensor signal. In some aspects, each of the plurality of buttons is associated with a unique signal threshold.

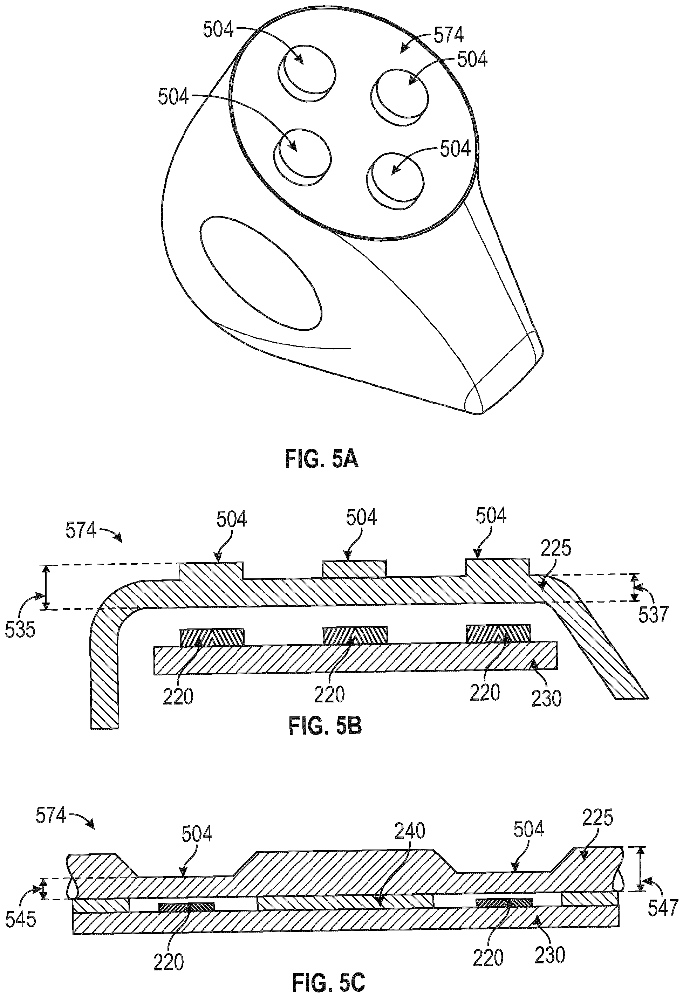

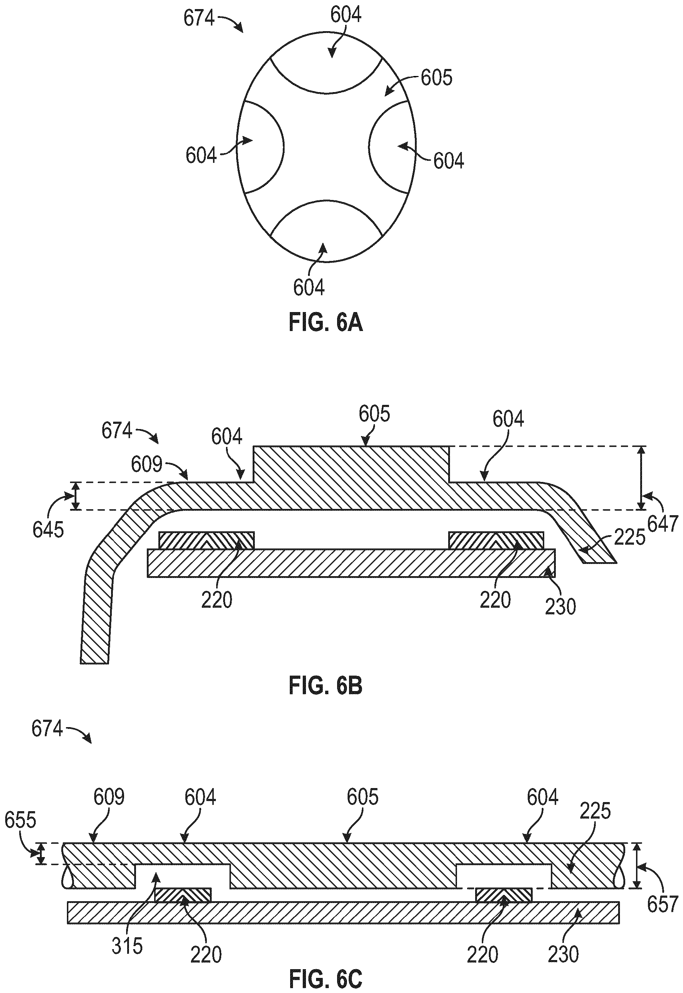

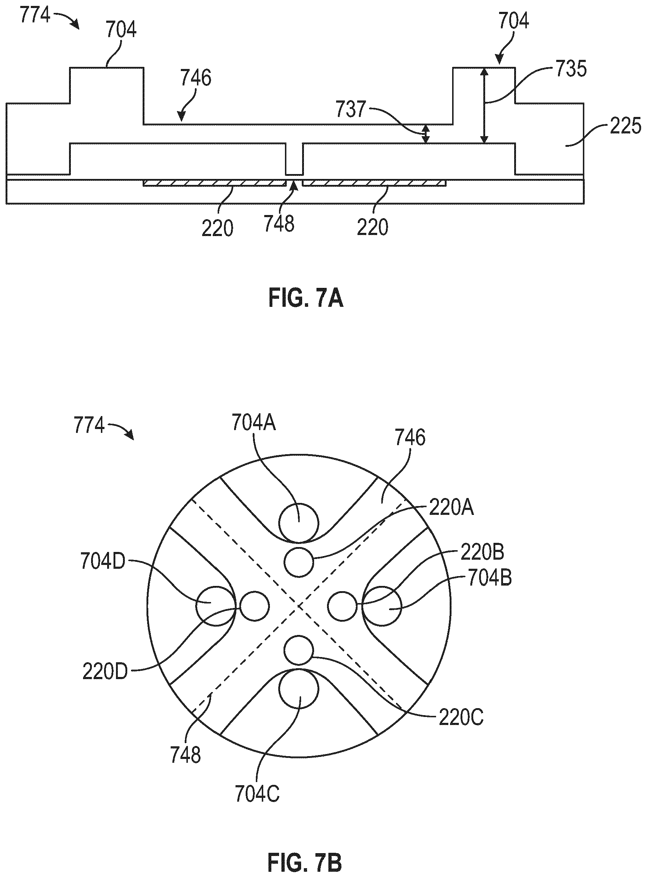

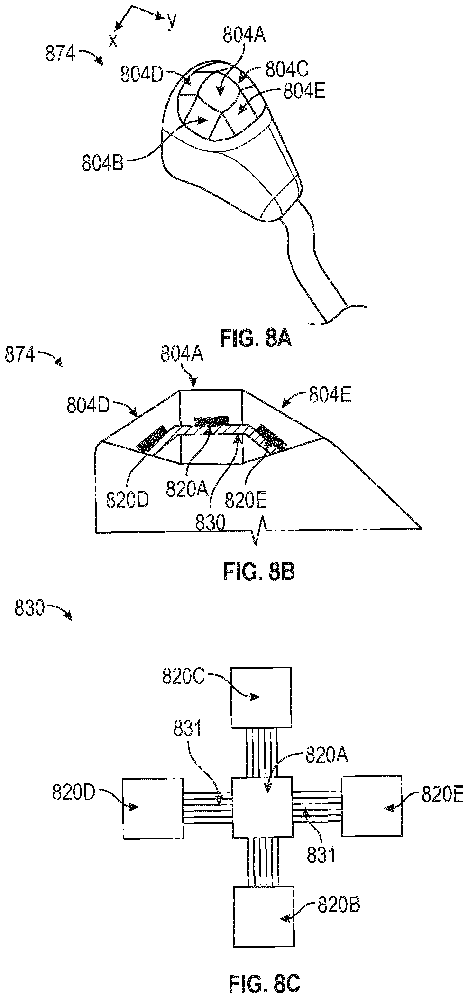

Aspects of the disclosure comprise keypad 174 (and/or other keypads disclosed herein such as keypad 574 of FIGS. 5A-5C, keypad 674 of FIGS. 6A-6C, keypad 774 of FIGS. 7A and 7B, or keypad 874 of FIG. 8A) including a tactile feedback device 234. The tactile feedback device 234 provides tactile feedback when a button press has been registered and/or when the button 204 is in its final position. The tactile feedback comprises one or more of a vibration, a click movement, and/or a sound. Example tactile feedback devices 234 include, but are not limited to, eccentric rotating mass (EMR) motors, linear resonant actuators (LRAs), piezo actuators, ultrasonic actuators, and solenoid actuators. In some aspects, the tactile feedback device 234 may be located anywhere within the camera head, not limited to locations under a button 204. In some aspects, the tactile feedback device 234 may be mechanically coupled to a body of the camera head such that energy is transferred from the tactile feedback device 234 to a button 204. In some aspects, the tactile feedback may be generated by a connected device, such as a sound emitted from a control unit 128, when a button press has been registered and/or when the button is in its final position.

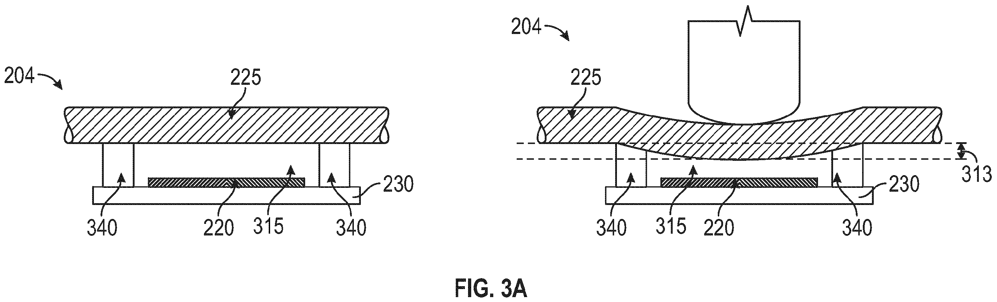

FIG. 3A illustrates cross-sectional views of a button 204 when the user is not pushing the button 204 (shown on the left) and when the user is pushing the button 204 (shown on the right). In some aspects, the user pushing the button 204 causes the material 225 to move by a deflection 313 due to the force from, *e.g*., the user's finger. The sensor 220 senses this deflection 313 and generates a sensor signal indicating this amount of deflection. In some aspects, the sensor 220 outputs the sensor signal indicating the amount of deflection of the material 225. The relationship between the sensor signal and the amount of deflection of the material 225 may be linear. For example, the sensor signal may be higher when the user is applying a higher amount of force on the material 225, thereby creating a larger amount of deflection 313. Conversely, the sensor signal may be lower when the user is applying a lower amount of force on the material 225, thereby creating a lower amount of deflection 313. In some aspects, the sensor signal is lowest when the button 204 is in the initial position (*e.g*., not pressed by the user), highest when in the final position (*e.g*., fully pressed by the user), and has a value between lowest and highest when in the intermediate position. For example, the sensor signal may be 0 V when the button 204 is in the initial position, 0.5 V when the button 204 is in the intermediate position, and 1 V when the button 204 is in the final position. In some aspects, the value of the sensor signal may be greater than the signal threshold when the button 204 is between the initial position and the final position.

As non-limiting examples, the sensor 220 comprises an inductive sensor, a capacitive sensor, a proximity sensor, or the like. Referring to the cross-sectional view of FIG. 3A, a spacer 340 may be located between the sensor 220 and the material 225 to ensure a uniform separation between the sensor 220 and the material 225. The button 204 may comprise air 315 or a suitable non-conductive material that is located between the sensor 220 and the material 225. The sensor 220, when an inductive sensor, can operate by way of an electromagnetic field directed at the material 225. The material 225 may interact with the electromagnetic field, causing a change in the sensor signal output by the sensor 220. In some aspects, the amount of deflection of the material 225 proximate to the sensor 220 may be associated with a strength of the electromagnetic field. An inductive sensor 220 may allow the keypad 174 to have a high resolution, capable of detecting microscopic deflections. The sensor 220, when a capacitive sensor, can operate based on capacitive coupling, where the amount of capacitive coupling can vary based on the distance between the sensor 220 and the material 225. In some aspects, the amount of deflection of the material 225 proximate to the sensor 220 may be associated with the amount of the capacitive coupling. The sensor signal output by the sensor 220 can indicate the amount of capacitive coupling.

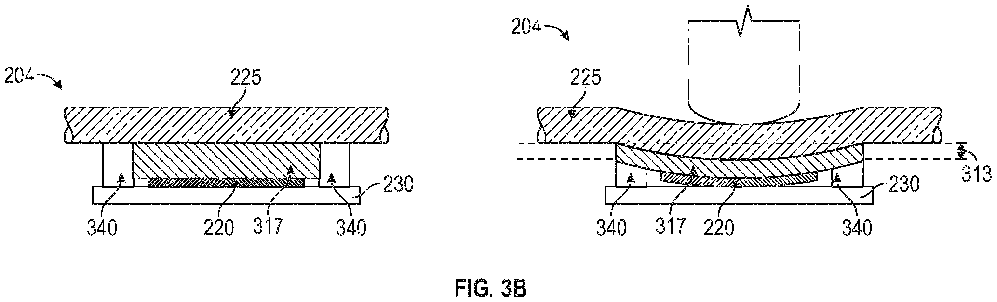

In some examples, the sensor 220 comprises a piezo sensor or a strain sensor. FIG. 3B illustrates cross-sectional views of a button 204 when the user is not pushing the button 204 (shown on the left) and when the user is pushing the button 204 (shown on the right). The button 204 comprises a material 317 that separates the sensor 220 and the material 225. The material 317 may comprise a material that is the same or different from material 225. Example materials for material 317 may include, but are not limited to, aluminum, stainless steel, carbon-reinforced polymer, polymer, titanium, and the like.

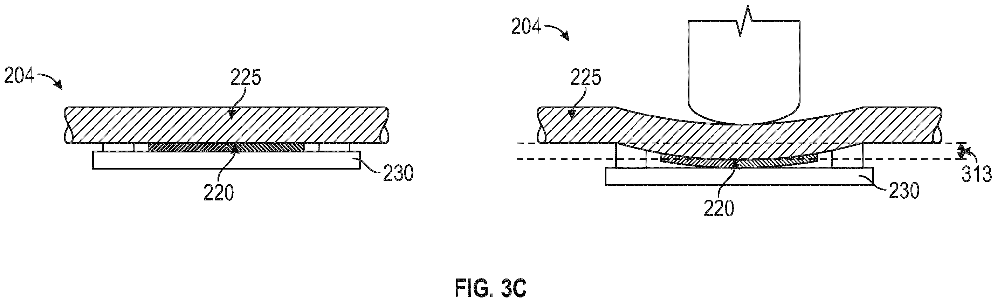

In some aspects, as shown in FIG. 3C (left shows user not pushing the button 204, and right shows user pushing the button 204), a material (such as material 317) may not be located between the material 225 and the sensor 220. The sensor 220, when a piezoelectric sensor, can operate by measuring a change in pressure (*e.g*., amount of stretching of the piezoelectric sensor) applied to the sensor 220, and outputting a sensor signal indicating the change in applied pressure. In some aspects, the amount of deflection of the material 225 proximate to the sensor 220 may be associated with an amount of stretching of the sensor 220. The sensor 220, when a strain sensor, can operate by converting the force applied to the sensor 220 (*e.g*., as measured by a change in resistance) into an electrical signal, which can be the sensor signal.

In some aspects, the keypad (*e.g*., keypad 174 of FIGS. 2A and 2B or other keypads disclosed herein such as keypad 574 of FIGS. 5A-5C, keypad 674 of FIGS. 6A-6C, keypad 774 of FIGS. 7A and 7B, and/or keypad 874 of FIG. 8A) is an endoscopic camera head keypad. The keypad may be located inside a camera enclosure. In some aspects, the buttons 204 (and/or other buttons disclosed herein such as buttons 504 of FIGS. 5A and 5B, buttons 604 of FIGS. 6A and 6B, buttons 704 of FIGS. 7A and 7B, buttons 804 of FIG. 8A, and/or buttons 805 of FIG. 8B) are located inside and/or integrated as part of the camera enclosure. For example, the buttons 204 can be formed by machining a continuous piece of material that forms the camera enclosure. That is, the material 225 forms both the buttons 204 and the camera enclosure. Using a single piece of material 225 for forming both the buttons 204 and the camera enclosure leads to reduced number of parts, lower costs, and a smaller camera head design.

In some aspects, circuitry for the camera head 173 (*e.g*., sensors 220, PCB 230, and corresponding wires) is also located in the camera enclosure. The camera enclosure may seal the internal wiring, enclosing the camera head 173 and allowing the camera head 173 (including the keypad 174) to be autoclavable. In some aspects, the camera enclosure is comprised of the material 225 capable of causing a deflection in response to a button press. By locating the buttons 204 and associated circuitry inside the camera enclosure, the keypad 174 can be hermetically sealed, thereby preventing or reducing leaks of, *e.g*., gas or liquids when autoclaving. In some aspects, there are no openings in the keypad 174; for example, no electronic feedthroughs that would have to be laser welded.

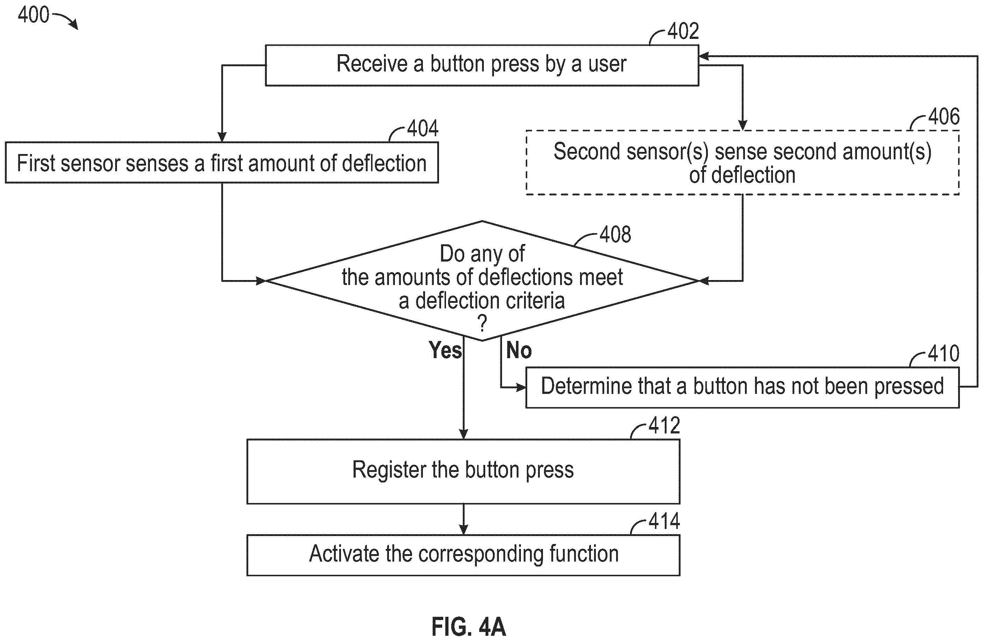

FIG. 4A illustrates a flowchart of an example method 400 for detecting a button press on a keypad 174 of a camera head 173, according to some aspects. The method 400 comprises receiving a button press by a user in step 402. In step 404, a first sensor 220 senses a first amount of deflection. In step 406, optionally, one or more second sensors 220 sense one or more second amounts of deflection. In some aspects, step 404 may be performed at the same time as step 406. In step 408, a controller determines whether any of the sensor signals from the sensors 220 indicating amounts of deflections has met a deflection criteria. For example, the controller identifies a value of the sensor signal being greater than a signal threshold, such as 70% of the maximum deflection, indicating a button press. If the deflection criteria are not met, then at step 410, the controller determines that a button 204 has not been pressed. If the deflection criteria have been met, then the controller registers a button press (step 412) and activates the corresponding function (step 414). In some aspects, the controller registers a button press in step 412 when the button 204 has been pressed for a certain amount of time, e.g., to reduce accidental button presses (where the button 204 may not be pressed for a long time). When the controller identifies a value of the sensor signal as greater than the signal threshold, the controller starts a timer and registers the button press when the elapsed time of the timer is greater than a time threshold.

In some aspects, the signal threshold may be a voltage threshold, a current threshold, a frequency threshold, a capacitance threshold, a strain threshold, etc. In some aspects, the signal threshold may be based on a position of the button 204, such as an initial position, a final position, a predetermined distance from the initial position, a predetermined distance from the final position, etc.

Aspects of the disclosure comprise an endoscopic system 120 capable of receiving different types of button presses, such as a short press and a long press. Different button presses may be used for different functions, for example. The controller may distinguish between the different types of presses based on the duration of when the deflection criteria is met.

A short press may be registered when the controller identifies a value of the sensor signal being greater than a signal threshold (first identification) and identifies a second value of the sensor signal being less than a second signal threshold (second identification). When the duration between the first identification and the second identification is shorter or longer than a duration threshold, then the controller may register the button press based on the type of button press that corresponds with the duration threshold. For example, the controller may register the button press as a short press when the duration between the first identification and the second identification is shorter than a short press duration threshold (*e.g*., 370 milliseconds). In some aspects, the controller determines whether or not the button press is a short press when the button is released. When the controller registers the button press as a short press, the camera head 173 performs a corresponding function, such as taking a picture.

As another example, the controller may register the button press as a long press when the duration between the first identification and the second identification is longer than a short press duration threshold (*e.g.,* 370 milliseconds) or longer than a long press duration threshold (*e.g*., 470 milliseconds). In some aspects, the controller determines whether or not the button press is a long press when the button is released. When the controller registers the button press as a long press, the camera head 173 performs a corresponding function. One example function that is activated with a long press is recording a video.

In some aspects, the deflection criteria may be met for multiple sensors 220. The controller may select among the buttons 204 whose deflection criteria have been met, register the corresponding button press, and activate the corresponding function. In some aspects, the controller may determine that the multiple button presses were intentional and may activate a corresponding function. In some aspects, a function may be activated when multiple buttons are pressed simultaneously (a multi-button press). For example, a first button may be associated with a first sensor signal and a second button may be associated with a second sensor signal. The controller may compare the first sensor signal to a signal threshold, and compare the second sensor signal to a signal threshold (same or different than the signal threshold used in the first sensor signal comparison). Based on these comparisons, the controller may determine whether the button press corresponds to a button press of a first button, a second button, or a multi-button press.

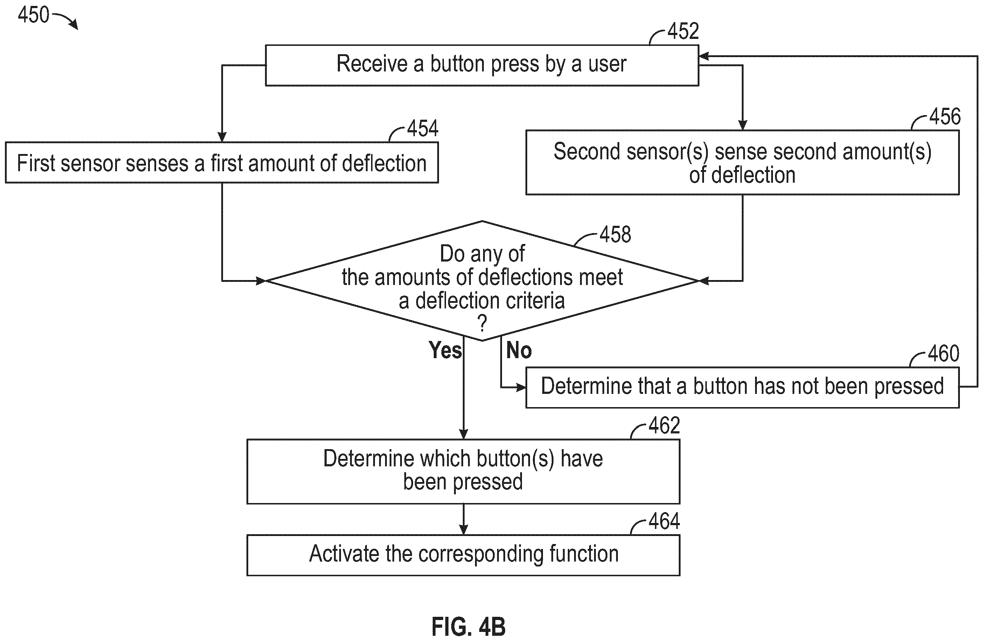

FIG. 4B illustrates a flowchart of an example method 450 for detecting multiple button presses on a keypad 174 of a camera head 173, according to some aspects. The method 450 comprises receiving a button press by a user in step 452. In step 454, a first sensor 220 senses a first amount of deflection, and in step 456, one or more second sensors 220 sense one or more second amounts of deflection. Step 454 may be performed at the same time as step 456, for example. In step 458, a controller determines whether any of the sensor signals from the sensors 220 indicating amounts of deflection has met a deflection criteria. The controller may determine that multiple buttons have met a deflection criteria. The controller, at step 462, may determine which of the button(s) 220 have been pressed, and then, at step 464, may activate the corresponding function. For example, a first sensor signal from a first button 204 may have 90% deflection, a second sensor signal from a second button 204 may have a 75% deflection, and a third sensor signal from a third button 204 may have a 30% deflection. The controller may determine that the first button 204 (90% deflection, for example) has been pressed. In some aspects, the controller may also determine that the second and third buttons (75% and 30% deflections, respectively, for example) have not been pressed. In some aspects, the controller may determine that the first and second buttons (90% and 75% deflections, respectively, for example) have been pressed, but the third button (30% deflection, for example) has not been pressed. If none of the amounts of deflections meet a deflection criteria, then the controller may determine that a button has not been pressed (step 460).

In some aspects, the controller may determine whether or not to register a button press based on the amount of time that the deflection criteria have been met. For example, if the user is tapping a button 204, the amount of deflection may meet the deflection criteria for a first amount of time. If the user is holding down a button 204, the amount of deflection may meet the deflection criteria for a second amount of time, where the second amount of time may be greater than the first amount of time. The controller may determine whether the amount of time (that the deflection criteria has been met) meets a time threshold, and if so, register the button press. In some aspects, the amount of time that the deflection criteria have been met may be determined based on the elapsed time since the start of a timer. The timer may start when the value of the sensor signal value is greater than the signal threshold. In some aspects, different buttons on the keypad 174 may have different time thresholds. For example, the buttons 204 of keypad 174 may be associated with a first time threshold, and a side button (*e.g*., button 805 shown in FIGS. 8A and 8B and discussed in more detail below) may be associated with a second time threshold.

Aspects of the disclosure comprise different configurations and/or geometries for a keypad. FIGS. 5A and 5B illustrate planar and cross-sectional views of an example keypad comprising raised buttons 504, according to some aspects. Keypad 574 comprises buttons 504, sensors 220, material 225, and PCB 230. The buttons 504 comprise one or more raised features that may, *e.g.,* help the user identify the locations of the buttons 504. The raised features may be formed as part of the material 225. The raised features may correspond to the buttons 504. In some aspects, the top surfaces of the raised features may be located further from the PCB 230 than the top surface at the locations of the keypad 574 comprising non-raised features. As shown in the cross-sectional view of FIG. 5B, the material 225 may comprise multiple thicknesses. For example, the material 225 may have a thickness 535 at locations of the raised features, where the thickness 535 of the material 225 at locations of one or more raised features may be greater than the thickness 537 of the material 225 at locations of non-raised features. The thickness 535 and/or 537 (and other thicknesses disclosed throughout) can range from 0.01-0.05 inches, for example.

In some aspects, the buttons 504 comprise recessed features, such as shown in the cross-sectional view of FIG. 5C, corresponding to one or more buttons 504. The recessed features may help the user identify the locations of the buttons 504. There may be less material 225 located at the recessed features than at locations of non-recessed features. That is, the thickness 545 (of material 225 at locations of the recessed features) may be less than the thickness 547 (of material 225 at locations of the non-recessed features).

FIGS. 6A and 6B illustrate top and cross-sectional views, respectively, of an example keypad comprising different regions of different thicknesses, according to some aspects. For example, the keypad 674 may comprise buttons 604 located at regions where the material has a thickness 645 that is less than the thickness 647 of the material at region(s) 605 surrounding the buttons 604. The thicker material 225 at surrounding region(s) 605 may help avoid or reduce accidental button presses, requiring a higher amount of force to cause a deflection of the material 225. In some aspects, the top surface 609 of the keypad 674 (or keypad 574 of FIG. 5B) may not be flat.

In some aspects, as shown in the cross-sectional view of FIG. 6C, the keypad 674 may have a top surface 609 (*e.g.,* surface where the user's finger would apply a force) that is flat, while also having different regions of different thicknesses. As shown in the figure, the thickness 655 of the material 225 at locations of the buttons 604 may be less than the thickness 657 of the material 225 at region(s) 605 surrounding the buttons 604. In some aspects, the sensor 220 and the material 225 may be separated by air 315 at the buttons 604 regions, and the material 225 may be separated by a spacer at surrounding region(s) 605. In some aspects, the material 225 may contact the PCB 230 at one or more surrounding regions located at the outer perimeter of the keypad 674, where there may not be any spacer located between the material 225 and the PCB 230. The separation distance between material 225 and sensor 220 may be less when utilizing the configuration of FIG. 6C (different regions of different thicknesses, where the top surface 609 is flat), which may allow more sensitive button detection due to the material 225 being closer to the sensor 220.

Aspects of the disclosure may comprise sensors that are located under the buttons, such as shown in FIG. 5C (sensors 220 are located under buttons 504) and as shown in FIG. 6C (sensors 220 are located under buttons 604). Examples of the disclosure also comprise sensors located proximate to the locations under the buttons. FIGS. 7A and 7B illustrate cross-sectional and top views, respectively, of an example keypad 774 comprising sensors located proximate to the location under the buttons, according to some aspects. Keypad 774 comprises buttons 704, sensors 220, and material 225. The sensors 220 may be located proximate to the locations under the buttons 704 (*e.g*., the sensors 220 may not be located under the buttons 704). As shown in the figure, the material 225 may comprise multiple thicknesses. For example, the material 225 may have a thickness 735 corresponding to the button 704 and a thickness 737 at a location above a sensor 220. In some aspects, the thickness 735 corresponding to the button 704 may be greater than the thickness 737 of the material 225 at locations above the sensors 220. The sensors 220 may sense the deflection of the material 225 at a deflection area 746. Although FIG. 7A illustrates a cross-sectional view of keypad 774, hatching has been omitted from the figure (*e.g*., for material 225) for purposes of clarity only.

In some aspects, the deflection area 746 may be at a location adjacent to a button 704. By sensing the material deflection at a location adjacent to the button 704 (instead of sensing the material deflection of the button 704), the keypad 774 may be configured with taller buttons 704 (greater thickness 735 of material 225). The taller buttons and/or greater thickness 735 of the material 225 may advantageously result in improved button differentiation (being able to differentiate between different buttons). Improving button differentiation may be useful in multiple situations, such as when the user is wearing gloves. The user may be able to wear gloves and differentiate among multiple buttons based on the feel of the buttons (*e.g*., without needing to look at the buttons 704 in order to find a button 704), or the user may be able to differentiate among multiple buttons 704 when not wearing gloves. The taller buttons and/or thicker material may allow the user to interact with the buttons 704 on the keypad 774 using touch and/or memory of the positions of the buttons 704. Additionally or alternatively, in some aspects, the material 225 may comprise a divider 748 configured to provide mechanical support that prevents over deflection of the material 225 (*e.g*., prevents the material 225 from bending too much/over bending when a button 704 is pressed).

The sensor 220 may be a capacitive sensor, for example, that can operate based on the amount of deflection of the material 225 above the sensor 220. Pressing the button 704 may cause the distance between the sensor 220 and the material 225 (above the sensor 220) to change. The distance may change the amount of deflection of the material 225, associated with the amount of capacitive coupling. For example, the sensor 220 may sense the deflection of a button 704 proximate to the sensor 220, and a button press may be registered as a result. In some aspects, the sensors 220 are located under the deflection area 746. Adjacent sensors 220 may be spaced such that a sensor 220 does not detect deflection of the material 225 above a different sensor 220 and/or material corresponding to a different button 704.

As shown in the top view of keypad 774 in FIG. 7B, the top of the keypad 774 may be partitioned into multiple buttons 704 with sensors 220 being uniformly distributed (when viewed from the top view, as shown in FIG. 7B) along the keypad 774. In some aspects, the keypad 774 may comprise one sensor 220 for each button 704. For example, as shown in FIG. 7B, sensor 220A may be configured to sense deflection of material 225 proximate to the button 704A (in a deflection area 746); sensor 220B may be configured to sense deflection of material 225 proximate to the button 704B; sensor 220C may be configured to sense deflection of material 225 corresponding to button 704C; and sensor 220D may be configured to sense deflection of material 225 corresponding to button 704D. In other aspects, the sensors 220 may be nonuniformly distributed (when viewed from the top view) along the keypad 774. Additionally or alternatively, although FIG. 7B illustrates four sensors 220 where there is one sensor 220 corresponding to a button 704, examples of the disclosure comprise any number of sensors 220 corresponding to a given button 704. That is, FIG. 7B illustrates one sensor 220 within each partition (divided according to the divider 748), but examples of the disclosure may include each partition comprising any number of sensors 220 suitable for detection a deflection of material 225.

FIGS. 8A and 8B illustrate planar and cross-sectional views of an example keypad 874 comprising buttons 804 with top surfaces on a plurality of planes, according to some aspects. For example, a first button 804A may be located on a top plane (whose first surface is oriented at a first angle of, *e.g*., 0°), a second button 804B may have a second surface oriented at a second angle (*e.g*., -45° along the x-axis) relative to the first surface of the first button 804A, a third button 804C may have a third surface oriented at a third angle (*e.g*., 45° along the x-axis) relative to the first surface of the first button 804A, a fourth button 804D may have a fourth surface oriented at a fourth angle (*e.g*., -45° along the y-axis) relative to the first surface of the first button 804A, and a fifth button 804E may have a fifth surface oriented at a fifth angle (*e.g.,* 45° along the y-axis). The first button 804A, second button 804B, third button 804C, fourth button 804D, and fifth button 804E may be formed of a single, continuous material 225. As shown in FIG. 8B, the keypad 874 may comprise a plurality of sensors 820. For example, one sensor 820 may be located under each button 804. In some aspects, the sensors 820 may be located on a plurality of planes, such as, a sensor 820 oriented parallel to the surface of a respective button 804, as shown in FIG. 8B. In some aspects, the plurality of planes comprises planes that differ from one another, and optionally, form a geodesic dome.

FIG. 8C illustrates a top view of an example PCB 830 comprising the sensors 820 for the keypad 874, in according to some aspects. The PCB 830 also comprises electrical wires 831 for electrically coupling the sensors 820 and communicating signals (*e.g*., comprising sensor signals output from the sensors 820) to a controller. The controller may be a hardware component located on one or more of the PCB regions that a sensor 820 is located on, or may be located on a separate PCB region. In some aspects, the PCB 830 may be a rigid flex PCB. The sensors 820 may be oriented relative to the top surfaces of the buttons 804 (as discussed above). In some aspects, the PCB regions may be located on different planes, forming a geodesic dome.

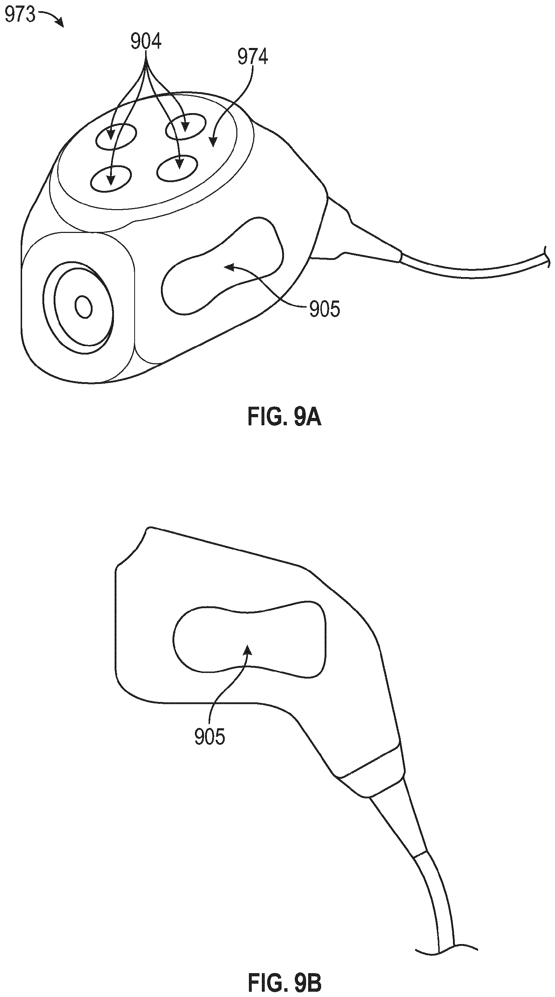

Aspects of the disclosure may comprise a camera head comprising a side button, such as illustrated in the planar and side views of FIGS. 9A and 9B, respectively. Camera head 973 may comprise a keypad 974, *e.g.,* located on the top of the camera head 973, and one or more side buttons 905, *e.g.,* located on one or more sides of the camera head 973. The side button 905 may comprise a sensor 220 (not shown), and the controller may detect button presses in accordance with the button press detection methods disclosed herein. In some aspects, the controller may be configured to sense an applied force to the side button 905, and register the applied force as a button press. For example, one or more side buttons 905 may be located on one or more sides of the camera head 973, where the user may push (*e.g*., squeeze) the side(s) of the camera head 973 for a button press. In some aspects, as shown in FIG. 9A, the keypad 974 may comprise a plurality of top buttons 904, which may be one or more of buttons 204, 504, 604, and/or 704. In some aspects, the side button 905 may be located close to a handle of the camera head 973, and aspects of the disclosure include registering a button press when the side button 905 is held down or squeezed. A button press of the side button 905 may be associated with a second time threshold (for registering a button press), which may be longer than a first time threshold associated with a button press of the buttons 904 on the keypad 974. In some aspects, the side button 905 may activate dedicated functions, such as activating voice commands or taking a picture, changing fluorescence modes, etc.

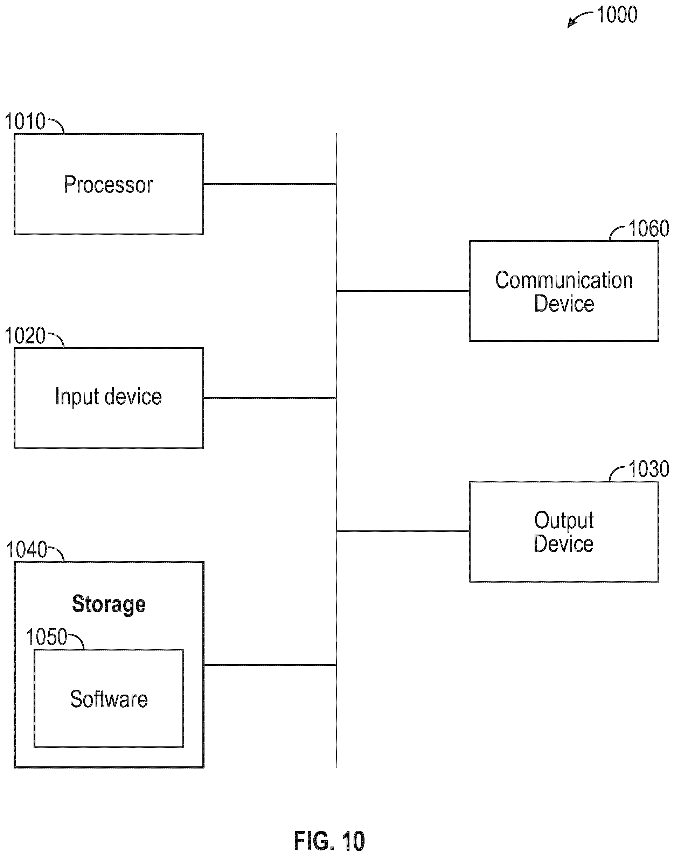

FIG. 10 illustrates an example computing system, in accordance with some examples, that can be used for performing any of the methods described herein, including method 400 of FIG. 4A or method 450 of FIG. 4B, and can be used for any of the systems described herein, including the endoscopic system 120 of FIG. 1, a camera head 173 comprising keypad 174 of FIGS. 2A and 2B, keypad 574 of FIGS. 5A-5C, keypad 674 of FIGS. 6A-6C, keypad 774 of FIGS. 7A and 7B, keypad 874 of FIGS. 8A and 8B, and/or keypad 1074 of FIG. 10A. System 1000 can be a computer coupled to a network, which can be, for example, an operating room network or a hospital network. System 1000 can be a client computer or a server. As shown in FIG. 10, system 1000 can be any suitable type of controller (including a microcontroller) or processor (including a microprocessor) based system, such as an embedded control system, personal computer, workstation, server, or handheld computing device (portable electronic device) such as a phone or tablet. The system can include, for example, one or more of processor 1010, input device 1020, output device 1030, storage 1040, or communication device 1060.

Input device 1020 can be any suitable device that provides input, such as a touch screen, keyboard or keypad, mouse, gesture recognition component of a virtual/augmented reality system, or voice-recognition device. Output device 1030 can be or include any suitable device that provides output, such as a touch screen, haptics device, virtual/augmented reality display, or speaker.

Storage 1040 can be any suitable device that provides storage, such as an electrical, magnetic, or optical memory including a RAM, cache, hard drive, removable storage disk, or other non-transitory computer readable medium. Communication device 1060 can include any suitable device capable of transmitting and receiving signals over a network, such as a network interface chip or device. The components of the computer can be coupled in any suitable manner, such as via a physical bus or wirelessly.

Software 1050, which can be stored in storage 1040 and executed by processor 1010, can include, for example, the programming that embodies the functionality of the present disclosure (*e.g*., as embodied in the devices as described above). For example, software 1050 can include one or more programs for performing one or more of the steps of the methods disclosed herein.

Software 1050 can also be stored and/or transported within any non-transitory computer-readable storage medium for use by or in connection with an instruction execution system, apparatus, or device, such as those described above, that can fetch instructions associated with the software from the instruction execution system, apparatus, or device and execute the instructions. In the context of this disclosure, a computer-readable storage medium can be any medium, such as storage 1040, that can contain or store programming for use by or in connection with an instruction execution system, apparatus, or device.

Software 1050 can also be propagated within any transport medium for use by or in connection with an instruction execution system, apparatus, or device, such as those described above, that can fetch instructions associated with the software from the instruction execution system, apparatus, or device and execute the instructions. In the context of this disclosure, a transport medium can be any medium that can communicate, propagate or transport programming for use by or in connection with an instruction execution system, apparatus, or device. The transport readable medium can include, but is not limited to, an electronic, magnetic, optical, electromagnetic, or infrared wired or wireless propagation medium.

System 1000 may be coupled to a network, which can be any suitable type of interconnected communication system. The network can implement any suitable communications protocol and can be secured by any suitable security protocol. The network can comprise network links of any suitable arrangement that can implement the transmission and reception of network signals, such as wireless network connections, T1 or T3 lines, cable networks, DSL, or telephone lines.

System 1000 can implement any operating system suitable for operating on the network. Software 1050 can be written in any suitable programming language, such as C, C++, C#, Java, or Python. In various examples, application software embodying the functionality of the present disclosure can be deployed in different configurations, such as in a client/server arrangement or through a Web browser as a Web-based application or Web service, for example.

The foregoing description, for the purpose of explanation, has been described with reference to specific aspects. However, the illustrative discussions above are not intended to be exhaustive or to limit the invention to the precise forms disclosed. Many modifications and variations are possible in view of the above teachings. The aspects were chosen and described in order to best explain the principles of the techniques and their practical applications. Others skilled in the art are thereby enabled to best utilize the techniques and various aspects with various modifications as are suited to the particular use contemplated.

Although the disclosure and examples have been fully described with reference to the accompanying figures, it is to be noted that various changes and modifications will become apparent to those skilled in the art. Such changes and modifications are to be understood as being included within the scope of the disclosure and examples as defined by the claims. Finally, the entire disclosure of the patents and publications referred to in this application are hereby incorporated herein by reference.

## Claims

1. A system comprising a camera head, the system comprising:
- a button;
- a material;
- a sensor configured to output a sensor signal indicating an amount of deflection of the material proximate to the sensor; and
- a controller configured to identify a value of the sensor signal being greater than a signal threshold, wherein the value of the sensor signal being greater than the signal threshold indicates a button press.

2. The system of claim 1, wherein the sensor is an inductive sensor, a capacitive sensor, or a piezo sensor.

3. The system of claim 1, wherein the controller is further configured to:
register the button press in accordance with the value of the sensor signal being greater than the signal threshold, alternatively
wherein the controller is further configured to:
- start a timer in accordance with the identification of the value of the sensor signal being greater than the signal threshold; and
- register the button press in accordance with an elapsed time of the timer being greater than a time threshold, alternatively,
wherein the controller is further configured to:
- determine a position of the button based on the sensor signal, wherein the position of the button comprises an initial position and a final position; and
wherein the value of the sensor signal being greater than the signal threshold occurs in accordance with the button being between the initial position and the final position, alternatively
wherein the controller is further configured to:
- identify a second value of the sensor signal being less than a second signal threshold, wherein the second value of the sensor signal being less than the second signal threshold indicates a button release, alternatively
wherein the controller is further configured to:
- identify a second value of the sensor signal being less than a second signal threshold; and
- register the button press as a short press in accordance with a duration being shorter than a short press duration threshold, wherein the duration is between the identification of the value of the sensor signal being greater than the signal threshold and the identification of the second value of the sensor signal being less than the second signal threshold, or alternatively
wherein the controller is further configured to:
- identify a second value of the sensor signal being less than a second signal threshold; and
- register the button press as a long press in accordance with a duration being longer than a long press duration threshold, wherein the duration is between the identification of the value of the sensor signal being greater than the signal threshold and the identification of the second value of the sensor signal being less than the second signal threshold.

4. The system of claim 1, further comprising:
a plurality of buttons, wherein each of the plurality of buttons is associated with one or more of: a unique sensor signal and a unique signal threshold.

5. The system of claim 1, wherein the system comprises a first button associated with a first sensor signal and a second button associated with a second sensor signal, wherein the controller further is configured to:
- compare the first sensor signal to the signal threshold;
- compare the second sensor signal to the signal threshold; and
- determine whether the button press corresponds to the first button, the second button, or a multi-button press based on the comparisons.

6. The system of claim 1, wherein the system comprises a first button associated with a first sensor signal and a second button associated with a second sensor signal, wherein the controller further is configured to:
- compare the first sensor signal to the signal threshold;
- compare the second sensor signal to a second signal threshold; and
- determine whether the button press corresponds to the first button, the second button, or a multi-button press based on the comparisons.

7. The system of claim 1, wherein the amount of deflection is between 5-200 microns.

8. The system of claim 1, wherein the material comprises one or more raised features or one or more recessed features corresponding to one or more buttons, and wherein a thickness of the material at locations of the one or more raised features or the one or more recessed features is greater than a thickness of the material at locations of non-raised features or non-recessed features.

9. The system of claim 1, further comprising:
a keypad comprising a plurality of buttons located on a plurality of planes, wherein the plurality of planes comprises different planes that form a geodesic dome.

10. The system of claim 1, wherein the button is associated with a first time threshold, the system further comprising:
- a side button associated with a second time threshold, wherein the second time threshold is longer than the first time threshold.

11. A method of detecting a button press of a button on a camera head, the method comprising:
- receiving a sensor signal from a sensor, wherein the sensor signal indicates an amount of deflection of a material proximate to the sensor;
- identifying a value of the sensor signal being greater than a signal threshold, wherein the value of the sensor signal being greater than the signal threshold indicates the button press; and
- registering the button press in accordance with the value of the sensor signal being greater than the signal threshold.

12. The method of claim 11, wherein the amount of deflection of the material is associated with one or more of: a strength of an electromagnetic field of the sensor, an amount of capacitive coupling, and an amount of stretching of the sensor.

13. The method of claim 11, further comprising:
- starting a timer in accordance with the identification of the value of the sensor signal being greater than the signal threshold; and
- registering the button press in accordance with an elapsed time of the timer being greater than a time threshold.

14. The method of claim 11, further comprising:
- determining a position of the button based on the sensor signal, wherein the position of the button comprises an initial position and a final position; and
wherein the value of the sensor signal being greater than the signal threshold occurs in accordance with the button being between the initial position and the final position.

15. An apparatus comprising:
- a button;
- a material;
- a sensor configured to output a sensor signal indicating an amount of deflection of the material proximate to the sensor; and
- a non-transitory computer-readable medium encoding instructions which, when executed by a processor, cause the processor to:
identify a value of the sensor signal being greater than a signal threshold, wherein the value of the sensor signal being greater than the signal threshold indicates a button press.
